# EUROPEAN PATENT SPECIFICATION

(11) **EP 3 009 430 B1**
(45) Date of publication and mention of the grant of the patent: **23.05.2018**
(21) Application number: 14189422.0
(22) Date of filing: 17.10.2014
(51) Int. Cl.: C07D 309/20

(54) **VINYLTETRAHYDROFURANES**
VINYLTETRAHYDROFURANE
VINYLTÉTRAHYDROFURANES

(43) Date of publication of application: 20.04.2016
(73) Proprietor: Henkel AG & Co. KGaA, 40589 Düsseldorf (DE)
(72) Inventor: KLANKERMAYER, Jürgen, 45259 Essen (DE); SCHLEKER, P. Philipp M., 52064 Aachen (DE); PALKOVITS, Regina, 52074 Aachen (DE); SANDBRINK, Lennart, 52134 Herzogenrath (DE)
(74) Representative: Strohe-Kamp, Geertje

(56) References cited:
- GABLE K P ET AL: "Kinetic Isotope Effects in Cycloreversion of Rhenium (V) diolates", JOURNAL OF THE AMERICAN CHEMICAL SOCIETY, AMERICAN CHEMICAL SOCIETY, vol. 124, no. 15, 1 January 2002 (2002-01-01), pages 3970-3979, XP002539126, ISSN: 0002-7863, DOI: 10.1021/JA017736W [retrieved on 2002-03-21]
- BOJANA SREO ET AL: "Heteroannelated ()-muricatacin mimics: synthesis, antiproliferative properties and structureactivity relationships", TETRAHEDRON, ELSEVIER SCIENCE PUBLISHERS, AMSTERDAM, NL, vol. 67, no. 48, 26 September 2011 (2011-09-26), pages 9358-9367, XP028326192, ISSN: 0040-4020, DOI: 10.1016/J.TET.2011.09.132 [retrieved on 2011-10-05]
- HON-CHUNG TSUI AND AL: "Reversible charge-accelerated oxy-cope rearrangements", JOURNAL OF ORGANIC CHEMISTRY, vol. 63, 1 January 1998 (1998-01-01), pages 9968-9977, XP002735009,
- HARI BABU MEREYALA AND: "Transformation of terminal diols of cyclic and acyclic saccharides to epoxides and alkylenes by reaction with triphenylphosphine,imidazole and iodine", JOURNAL OF CARBOHYDRATE CHEMISTRY, vol. 19, no. 9, 1 January 2000 (2000-01-01), pages 1211-1222, XP002735010,

## Description

The present invention relates to the field of vinyl compounds, especially vinyltetrahydrofuranes.

One of the biggest challenges of this century is the development of novel ways to harness renewable resources as an alternative to petroleum-based value chain. Both the energy and fuel sector as well as the chemistry sector with everyday chemicals such as plastics, paints, soaps, etc. are extremely dependent on oil. With the increasing demand and declining reserves of oil a change to a different raw material base will be necessary in future. As part of the ongoing industrialization of emerging economies in particular the extension of the value chain is more significant. Renewable resources have a completely different chemical composition compared to oil. The refinery established technologies such as thermal and catalytic cracking, isomerization and other petrochemical processes can therefore not be used directly. For this reason, new pathways and tools need to be developed and analyzed to establish analogous to a petroleum refinery, a biorefinery with specific technologies.

For this reason, it is an object to provide novel suitable building blocks and methods to their production. Especially of interest are vinyl- or alkene-containing materials due to their polymerizability which are on the other hand somewhat polar due to suitable functional groups.

This object is solved by the method of claim 1. Accordingly, a method of the production of (2R,3R,4S)-2-vinyltetrahydrofuran-3,4-diol is provided.

Methods for converting diols are inter alia known from Gable et al, JACS 2002, 3970, Sreo et al, Tetrahedron 2011, 9358, Tsui et al JOC 1998, 9968 and Mereyala et al, J. Carboh. Chem. 2000, 1211.

(2R,3R,4S)-2-vinyltetrahydrofuran-3,4-diol has, depending on the actual application, at least one or more of the following advantages:
- As will be shown later on, it can be produced easily from glucose and fructose, compounds abundant in biomass
- Due to the two hydroxy groups the compound is highly polar, making it an interesting candidate for many polymers
- The incorporated functionalities in the compound enable the chemical modification of surfaces and materials
- The incorporated functionalities in the compound enable the synthesis of surfactants

Yet accordingly, a method of producing (2R,3R,4S)-2-vinyltetrahydrofuran-3,4-diol is provided, comprising the step of reacting 1,4-sorbitan with a Rhenium-compound (Re-compound), selected from HReO₄, Re₂O₇, H₃CReO₃, AReO₄ with A is selected from a monovalent metal or ammonium ion, Re(CO)₅Br and Re₂(CO)₁₀ and mixtures thereof, in the presence of a protic solvent, which is a mono- or polyvalent alcohol, whereby the Re-compound is immobilized or present in heterogenous form.

Surprisingly it has been found that by this method the target compound (2R,3R,4S)-2-vinyltetrahydrofuran-3,4-diol can be obtained as the only stereoisomer. 1,4-Sorbitan itself can be made easily from glucose or fructose via literature-known methods.

The Re-compounds mentioned above have proven themselves in practice. Especially preferred are HRO₄, Re₂O₇ and H₃CReO₃, more especially HReO₄, since in many actual applications they have given the best yields.

However, this is not limiting to the present invention, and according to a preferred embodiment of the present invention, the Re-compound comprises Rhenium in a lower oxidation state than (VII), either as the active compound or as a precursor. Especially preferred compounds include Re(CO)₅Br (Rhenium (I)) and Re₂(CO)₁₀ (Rhenium (0)).

The Re-compound is present in heterogenous form. Preferably the Re-compound is bound to a solid carrier, such as - but not limited to - active coal, functionalized active coal, polymers, especially hyperbranched polymers, and oxidic materials, including TiO₂, ZrO₂, SiO₂, zeolite, Al₂O₃, Co₃O₄.

Precursor materials for immobilizing the Re-compound may be selected from NH₄ReO₄, Re₂O₇, HReO₄, ReO₂, ReO₃, Re, H₃CReO₃ and Re₂(CO)₁₀.

Preferred methods for immobilizing the Re-compound include wet impregnation, dry impregnation, absorption or precipitation.

According to a preferred embodiment of the present invention, the protic solvent has a boiling point of 130°C or more.

Preferred solvents are especially 1-octanol and/or 2-octanol.

According to a preferred embodiment of the present invention, the method is carried out without any further reagents. This has shown to be advantageous and side-reaction prohibiting for many applications within the present invention.

According to a preferred embodiment of the present invention, the method is carried out at a temperature of ≥100°C.

The aforementioned components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the subclaims and the following description of the respective examples --which in an exemplary fashion-- show one preferred embodiment of the rotary drum according to the invention. Such embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

### EXAMPLE I:

### Preparation of (2R,3R,4S)-2-vinyltetrahydrofuran-3,4-diol:

Under an argon atmosphere 1,4-sorbitan (606.6 mg, 3.70 mmol), HRe04 (aq.)(65w%) (33.1 µL, 46.4 mg, 184.7 µmol, 5 mol%) and 24.3 ml 2-octanol were given in a bottle flask with condenser. About 7.0 g Molecular sieve (4Å) are placed on a sieve between Flask and condenser. The solution was heated to 170 °C in an oil bath and stirred for 2 hours.

The catalyst was removed from the reaction mixture by silica column chromatography (Ethyl acetate).

Ethyl acetate was removed by rotation evaporation. The resulting solution was extracted with water/methanol (1.5/1.1). Water and methanol was removed from the resulting solution and the remaining high viscous oil was again purified by column chromatography. The product fraction was dried and subsequently sublimated (60°C, high vacuum).

### Product: 255.8 mg (2R,3R,4S)-2-vinyltetrahydrofuran-3,4-diol:

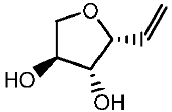

¹H-NMR (600 MHz, MeOH-d₄): δ = 3.65 (dd, J =9.3 Hz, 1.1Hz; 1H), 3.93 (dd, J=3.2 Hz, 1.1Hz; 1H), 4.14 (dd, J=9.3Hz, 4.3Hz; 1H), 4.17 (m, 1H), 5.25 (ddd, J=10.4Hz, 1.9Hz, 1.0Hz; 1H), 5.36 (ddd, J=17.3, 1.3Hz, 1.9Hz; 1H), 5.95 (ddd, J=17.3Hz, 10.4Hz, 7.0Hz; 1H) ppm.
¹³C-NMR (151 MHz, MeOH-d₄): δ =74.4, 78.5, 79.6, 83.7, 118.5, 135.1 ppm.
HRMS (ESI) found [M+Na]⁺:153.05219, C₆H₁₀O₃Na requires: 153.05222

## Claims

1. A method of producing (2R,3R,4S)-2-vinyltetrahydrofuran-3,4-diol, comprising the step of reacting 1,4-sorbitan with a Rhenium-compound (Re-compound), selected from HReO₄, Re₂O₇, H₃CReO₃, AReO₄ with A is selected from a monovalent metal or ammonium ion, Re(CO)₅Br and Re₂(CO)₁₀ and mixtures thereof, in the presence of a protic solvent, which is a mono- or polyvalent alcohol, whereby the Re-compound is immobilized or present in heterogenous form.

2. The method of claim 1, whereby the protic solvent has a boiling point of 130°C or more.

3. The method of claim 1 or 2, characterized that it is carried out without any further reagents.

4. The method of any of the claims 1 to 3, characterized that it is carried out at a temperature of ≥100°C.

## Patentansprüche

1. Verfahren zum Herstellen von (2R,3R,4S)-2-Vinyltetrahydrofuran-3,4-diol, umfassend den Schritt eines Umsetzens von 1,4-Sorbitan mit einer Rhenium-Verbindung (Re-Verbindung), ausgewählt aus HReO₄, Re₂O₇, H₃CReO₃, AReO₄, wobei A ausgewählt ist aus einem einwertigen Metall oder einem Ammoniumion, Re(CO)₅Br und Re₂(CO)₁₀ und Mischungen daraus, in der Gegenwart eines protischen Lösungsmittels, das ein einwertiger oder mehrwertiger Alkohol ist, wobei die Re-Verbindung immobilisiert oder in einer heterogenen Form vorhanden ist.

2. Verfahren nach Anspruch 1, wobei das protische Lösungsmittel einen Siedepunkt von 130 °C oder mehr aufweist.

3. Verfahren nach Anspruch 1 oder 2, **dadurch gekennzeichnet, dass** es ohne jegliche weitere Reagenzien ausgeführt wird.

4. Verfahren nach einem der Ansprüche 1 bis 3, **dadurch gekennzeichnet, dass** es bei einer Temperatur von ≥100 °C ausgeführt wird.

## Revendications

1. Procédé de production de (2R,3R,4S)-2-vinyltétrahydrofuran-3,4-diol, comprenant l'étape de mise en réaction de 1,4-sorbitane avec un composé de Rhénium (Composé Re), choisi parmi HReO₄, Re₂O₇, H₃CReO₃, AReO₄, A étant choisi parmi un ion métallique ou ammonium monovalent, Re(CO)₅Br et Re₂(CO)₁₀ et leurs mélanges, en présence d'un solvant protique, qui est un alcool mono- ou polyvalent, le composé Re étant immobilisé ou présent sous forme hétérogène.

2. Procédé selon la revendication 1, le solvant protique ayant un point d'ébullition supérieur ou égal à 130°C.

3. Procédé selon la revendication 1 ou 2, **caractérisé en ce qu'**il est exécuté sans aucun réactif supplémentaire.

4. Procédé selon l'une quelconque des revendications 1 à 3, **caractérisé en ce qu'**il est exécuté à une température supérieure ou égale à 100°C.
